# EUROPEAN PATENT APPLICATION

(11) **EP 3 744 252 A1**
(43) Date of publication of application: **02.12.2020**
(21) Application number: 18902601.6
(22) Date of filing: 07.05.2018
(51) Int. Cl.: A61B 5/16, A61M 21/00

(54) **METHOD FOR PROVIDING REMOTE PSYCHOLOGICAL HELP**

(30) Priority: 24.01.2018 RU 2018102742
(71) Applicant: Horoshutin, Pavel Pavlovich, G. Irkutsk 664009 (RU)
(72) Inventor: Horoshutin, Pavel Pavlovich, G. Irkutsk 664009 (RU)
(74) Representative: Vitina, Maruta
(86) International application number: PCT/RU2018/000295
(87) International publication number: WO 2019/147161

(57) **Abstract**

The invention relates to medicine, psychology, solving dependency issues, and correcting social behavior. A patient anonymously and independently conducts a psychological assessment and corrective psychological treatment using remote communication means, without contact with a psychologist, by means of testing. The sensory preferences of the patient are ascertained, and the number and type of sensory stimuli that elicit maximally pleasant sensations for the patient are determined for all five channels of sensory perception on the basis of said preferences. Based on the results, a set of materials is remotely assembled and made available for the patient to independently undergo a course of corrective treatment, including an autogenic training text, sensory stimuli, tools for the use of said stimuli, and instructions. The method provides individualized, simplified psychological aid, security, anonymity, and accessibility for people living in distant communities.

## Description

The invention relates to psychology, and specifically to a method of providing remote psychological aid for solving problems related to mental states and dependencies and for correcting problematic social behavior.

It is known that more than half of patients of psychologists experience stress before the first meeting with a specialist, and it is also known that direct contact of a psychologist with a patient in the provision of psychological aid often leads to the emergence of new forms of dependencies. Furthermore, there is the problem of providing psychological services to patients who live in remote settlements and have no opportunity to receive qualified psychological aid. The proposed method of providing remote psychological aid is provided to solve these problems, namely, to work with patients who need qualified psychological aid, but live in remote settlements, and with patients who want to receive such aid remotely, or even anonymously.

Known is a method of interactive treatment of addictive behavior (see the invention description of patent No. RU2252038, published in 2005, A61M21/00) comprising providing psychotherapeutic, suggestive impact on a patient, affecting by sound, setting the attitude related to the formation of negative emotions with a focus of the patient on unpleasant grotesquely emphasized moments of abuse by projecting, on a screen of audiovisual devices, a video sequence of negative pictures and forming positive emotions in the patient when showing positive pictures, where the psychotherapeutic, suggestive impact is provided through channels of audiovisual devices under constant remote control of psycho-emotional state of the patient and the level of addictive behavior attitude of the patient by the doctor, where initial psycho-physiological characteristics, fluctuations of the omega-potential, anxiety level, emotional reactions to relevant questions at rest and in presence of negative emotions are firstly registered, and a dominance for a healthy lifestyle and a program for abandoning the dependencies is formed in the patient in presence of positive emotions, while improving psycho-emotional state controlled according to stability of psycho-physiological reactions and reducing anxiety, performing correction of behavioral stereotypes with development of an adaptive self-realization strategy, where the patient by himself selects a motivating video image as a permanent screen saver of an audiovisual device to activate the previously formed dominance and the program for abandoning the dependencies, and the verbal impact is performed in a stereophonic mode with binaural sounds vibrations in the vocal range and an adjustable frequency difference for the right and the left channels from 30 to 3 Hz.

Known is an analogous technical solution, see the invention description of patent No. RU2319517, published in 2008, A61M21/00, which is selected to be the closest analogue, or the prototype, and includes registration of psycho-physiological reactions of a patient, selection of a remote individual program for interactive correction of addictive behavior of the patient in the presence of persons interested in refusal from addictive behavior, as well as audio and video information including the attitude related to the refusal from a subject of dependency, and preliminarily training the interested parties to assess a condition of the patient and perform a plan of action in case of withdrawal behavior of the patient, upon which, in case of withdrawal behavior, these persons use a mobile phone to dial a psychological service operator, where, at the beginning of treatment, firstly, transferring of audio information of the interactive correction program is initiated, followed by turning on the video information of the interactive correction program, and then, upon change of the level of addictive behavior of the patient, correction of the interactive program is performed at the request of the patient himself or interested persons.

The disadvantages of the known methods include the facts that the psychodiagnostics takes place upon direct contact of a doctor with a patient and, therefore, the situation for the primary stress occurrence still exists. The treatment process is further carried out remotely, but with constant monitoring and in direct contact with an attending physician, which may cause the emergence of new dependencies. The known methods provide no possibility for applying the anonymity principle.

In contrast to the known methods, the proposed method of providing remote psychological aid is aimed at providing qualified psychological aid in the absence of direct contact of a patient with a specialist by applying the anonymity principle while providing psychodiagnostics and psychological correction.

The technical effect, which is impossible to achieve by any of the above-mentioned methods, is to simplify the method of providing psychological aid in case of mental states and dependencies.

Based on the characteristics and analysis of the known analogous methods, it can be concluded that the problem of creating less complex methods of providing remote psychological aid is currently important.

The technical effect is achieved by the fact that the method of providing remote psychological aid includes psychodiagnostics of a mental state of a patient to resolve problems of behavior related to mental states and dependencies, in which long-distance communication means are used during the psychodiagnostics and psychological correction, in which the psychodiagnostics and the psychological correction of mental problems of the patient are performed without contacting a psychologist, an item kit required to undergo an autogenic training course by a patient himself is selected based on psychodiagnostics data individually for the patient, the item kit includes text of an autogenic training, sensor stimuli and tools for their use, an instruction on use, in which the kit of necessary items for conducting the autogenic training by the patient is remotely provided to the patient, the patient conducts the autogenic training by himself using items intended for conducting the autogenic training. The psychodiagnostics of the mental state of a patient is performed anonymously. The autogenic training course takes place twice a day, in the morning and in the evening, for at least 10 minutes for at least 1 week.

The proposed method of providing remote psychological aid is performed as follows. A patient is registered with a psychological center using a long-distance communication means (for example, a phone, Internet, mail, etc.), where the anonymous registration is possible. After that, the patient remotely performs psychodiagnostics using tests. The patient answers the test questions by himself. After receiving the results of psychodiagnostics, a psychologist develops an autogenic training course and selects necessary items for the implementation of the autogenic training course. The selected item kit and instructions for the use of items are sent to the patient, for example, by a delivery service. The patient undergoes the course of psychological correction by himself based on the instructions. If the patient has difficulties understanding the instructions, the patient may, for example, receive explanations using a long-distance communication means, for example, by phone, or by mail.

Sensory stimuli and a method of their selection are known, for example, from patents No. RU2315633 and No. RU2178204. A kit having sensor stimuli is described in patent No. 2632772. The principle of selection of items is the same.

The selection of an item kit based on the results of individual psychodiagnostics for the course of autogenic training can be carried out, for example, as follows.

In the course of the correction of psychological states and dependencies, a psychologist remotely tests a patient to determine individual characteristics of the human perception sensory system, thereby ascertaining the sensory preferences of the patient, namely items evoking pleasant impressions through seven information perception sensory channels. Based on the testing of the patient, a number of sensory stimuli and their appearance that will maximally evoke pleasant impressions are determined.

For example, the following item kit may be proposed.

A kit of essential oils and a device for their use, such as an aroma lamp. Particular essential oils are selected by the patient during remote psychodiagnostics and are used to create a persistent aroma in the room. This tool creates a pleasant impression through the **olfactory** sensory channel of information perception.

The text of autogenic training and an audio player for text listening. The text is recorded using the audio player by the voice of the patient by himself and is then listened to, for example, daily. It is directed to changing the attitude and creates a pleasant impression through the **auditory** sensory channel of information perception.

An image with a selected suggestive formula to change the attitude is an image made according to a design determined based on the psychodiagnostics, with key linguistic phrases of the suggestive formula. The patient chooses a particular image, as well as color and text font, which are the most pleasant to him, by himself during the psychodiagnostics. The suggestive formula is one or more linguistic phrases that form a new attitude. This tool includes the **visual** sensory channel of information perception into the autogenic training process.

An image with a formula of kinesthetic sensations. It is an image made according to a design determined based on the psychodiagnostics and including one or more linguistic phrases targeting kinesthetic sensations, the character of which is also determined during the testing. The patient chooses a particular image, as well as color and text font, which are the most pleasant to him, by himself during the psychodiagnostics. It creates pleasant impressions through the kinesthetic sensory channel of information perception. It includes the **kinesthetic** sensory channel of information perception into the autogenic training process.

An image with a formula of reasoned persuasions. It is an image made according to a design determined based on the psychodiagnostics. The patient selects the design and particular image from variants provided in tests by himself during the psychodiagnostics. It is based on linguistic phrases, which are also formed based on tests and focus the attention of the patient during the autogenic training on pleasant reasoning conclusions. It creates pleasant impressions through the reasoning sensory channel of information perception. It includes the **reasoning** sensory channel of information perception into the autogenic training process.

A food additive having a strong taste. It is a food additive pleasant for the patient, for example, lollipops, which cause a long pleasant taste effect. It is determined during the psychodiagnostics. It is selected by the patient from variants provided in tests. It is used by the patient while listening to the text of the autogenic training. It creates pleasant impressions through the **gustatory** sensory channel.

Minerals providing various tactile sensations. They are minerals of various shapes. They are selected by the patient from variants provided in tests during the psychodiagnostics. They are held by the patient in his hand while listening to the text of the autogenic training. They create a pleasant impression through the tactile sensory channel of information perception.

An instruction for use of items is a text explaining the order of application of the entire item kit during an individual autogenic training.

The technical essence of the proposed method of providing remote psychological aid includes the following:
- creating long-distance contactless communication channels between the patient and the psychologist via mail, phone, or Internet;
- performing psychodiagnostics of the mental state of the patient using tests;
- creating a program for conducting the autogenic training by the patient;
- drafting an individual text of an autogenic training directed to changing the attitude being the reason for undesirable dependencies or mental states or social behavior;
- selecting a kit of necessary items for conducting the autogenic training by the patient;
- sending the kit of necessary items for conducting the autogenic training to a location of the patient;
- providing repeated listening by the patient to the text of the autogenic training with the simultaneous use of items intended for conducting the autogenic training for at least one week every morning and every evening for at least 10 minutes;
- enhancing, on the subconscious level, the attitude relating to quitting addictive behavior.
- providing the process of psychological rehabilitation to the patient having mental dependencies and states and correction of social behavior;

The method of providing remote psychological aid may be performed in practice as follows:
- creating a long-distance communication channel between the patient who applied to a psychological center for help and a psychologist using, for example, a phone, mail, or the Internet;
- performing psychodiagnostics of the mental state of the patient and the causes of the problematic behavior of the patient related, for example, to alcohol addiction;
- determining a list of positive impacts (sound, smell, images, etc.) on the patient;
- developing a program for conducting an autogenic training by the patient, where the program includes a sequence of particular actions prescribed for performance by the patient and providing psychological correction of states and dependencies of the patient;
- drafting an individual text of an autogenic training directed to quitting problematic behavior by the patient;
- selecting a kit of necessary items for conducting the autogenic training by the patient, namely, the text of the autogenic training, stimuli for pleasant classical conditioning through all sensory channels of perception and tools for their use, an instruction for use.
- sending the kit of necessary items and the instructions for use to a location of the patient;
- administering are peated audio listening of the text of the autogenic training by the patient with the simultaneous use of items intended for conducting the autogenic training, for example, for two months every morning and every evening, for example, for 10 minutes;
- enhancing, on the subconscious level, the attitude associated with quitting the behavior being the reason for undesirable dependencies or mental states;

The implementation of the proposed method of remote psychological aid is illustrated by the following examples.

### Example No. 1. Psychological correction of dependencies.

A 35-year-old man having a nicotine addiction and desire to quit smoking applied for help. The man registered with the Internet site of the psychological center. After that, the man got remote access to a psychodiagnostics department and answered questions from 7 tests. Based on the answers of the man, information on the state of a sensory system of perception of the man was obtained, the attitude forming the basis for problematic behavior was determined, and sensory preferences were identified. The received information was used to develop an autogenic training course with full-sensory classical conditioning aimed at formation of the behavior free from nicotine addiction. The package named "An item kit for full-sensory psychological rehabilitation" included: an instruction for conducting the autogenic training, the text for self-suggestion, five sensory stimuli for pleasant classic conditioning, and tools for their use.

The sensor stimuli included:
- for the taste sensation channel: a flavored food additive being lollipops with mint flavor,
- for the aroma sensation channel: one fir essential oil and aromatic medallion for its use,
- for the tactile sensation channel: jade beads,
- for the sound sensation channel: a voice recorder and text of the autogenic training,
- for the visual perception channel: a table having a particular design with a suggestive formula,
the items were selected by the patient from several proposed options during testing as evoking the most pleasant impressions in the patient.

The package was sent to the patient by an express delivery service. The patient called once to get clarification on the order of using the items. No further contacts with the patient occurred. No complaints and negative reviews have been received on the site.

### Example 2. Psychological correction of states.

A 29-year-old woman applied for help. The reason for applying for help was the aggressive behavior in the family and conflicts with the husband. Upon registering on the site and passing the remote psychodiagnostics, a map of mental state of the woman was drawn up, causes of fear underlying the aggressive behavior were determined, and a psychological correction course was developed. Based on this, an item kit for remote psychological aid was formed and sent to the patient. Several calls were further received from the patient in which the patient asked for additional recommendations related to the autogenic training course. After three months, the patient left a positive review on the site.

### Example 3. Psychological correction of social behavior.

Upon publication of an announcement on provision of psychological services in a regional newspaper, a letter was received from a 58-year-old woman who requested psychological aid for her grandson, who did not want to go to school. Tests for psychodiagnostics were sent by letter to the address of the woman, for her grandson, and after they were received by the psychologist, a psychological correction program aimed at formation of a new type of behavior with a passion for learning was developed. A package with an item kit for full-sensory psychological rehabilitation was sent to the village. The grandson took the autogenic training course under the control of his grandmother, and, according to the woman, the grandson stopped skipping classes at school.

The proposed method of providing remote psychological aid provides preparing an individual autogenic training course for a patient and implementing of the autogenic training course by the patient himself without a psychologist, thereby enabling creating a safer, more convenient and less technically complex method of psychological correction of mental states and dependencies and correction of social behavior.

The introduction of a remote and anonymous way into the existing practice of psychological counseling will help to provide professional psychological aid to a category of people who currently avoid contacts with psychologists and psychotherapists due to the stress and provide services to patients living in remote settlements and having no opportunity to receive psychological aid.

## Claims

1. A method of providing remote psychological aid including psychodiagnostics of a mental state of a patient to resolve problems of behavior related to mental states and dependencies, wherein long-distance communication means are used during the psychodiagnostics and psychological correction, **characterized in that** the psychodiagnostics and the psychological correction of mental problems of the patient are performed without contacting a psychologist, an item kit required to undergo an autogenic training course by a patient himself is selected based on psychodiagnostics data for the patient, the item kit including text of an autogenic training, sensor stimuli and tools for their use, an instruction on use, wherein the kit of necessary items for conducting the autogenic training by the patient is remotely provided to the patient, the patient conducts the autogenic training by himself using items intended for conducting the autogenic training.

2. The method of providing remote psychological aid of claim 1, **characterized in that** the psychodiagnostics of the mental state of the patient is performed anonymously.

3. The method of providing remote psychological aid of claim 1, **characterized in that** the autogenic training course takes place twice a day, in the morning and in the evening, for at least 10 minutes for at least 1 week.

4. The method of providing remote psychological aid of claim 1, **characterized in that** the kit of items is sent to the patient by a delivery service or by mail.
